# FASCICULE DE BREVET EUROPEEN

(11) **EP 1 002 520 B1**
(45) Date de publication et mention de la délivrance du brevet: **29.10.2008**
(21) Numéro de dépôt: 99402648.2
(22) Date de dépôt: 25.10.1999
(51) Int. Cl.: A61K 8/41, A61K 8/49, A61Q 5/10

(54) **Composition de teinture d'oxydation des fibres kératiniques et procédé de teinture mettant en oeuvre cette composition**
Oxidationsfärbungszusammensetzung für keratinische Fasern und Färbungsverfahren mit derselben
Composition for oxidative dying of keratinous fibres and dying process therewith

(30) Priorité: 20.11.1998 FR 9814653
(43) Date de publication de la demande: 24.05.2000
(73) Titulaire: L'ORÉAL, 75008 Paris (FR)
(72) Inventeur: Audousset, Marie Pascale, 92600 Asnieres (FR)
(74) Mandataire: Fevrier, Murielle Françoise E.

(56) Documents cités:
- EP-A- 0 728 464
- EP-A- 0 832 640
- GB-A- 2 260 135

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, du 1,3-bis-(β-hydroxyéthyl)amino 2-méthyl benzène et/ou au moins l'un de ses sels d'addition avec un acide à titre de premier coupleur, et au moins un coupleur hétérocyclique sélectionné à titre de second coupleur, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet DE-A-4 132 615 et DE-A-19 637 371, des compositions pour la teinture d'oxydation des fibres kératiniques contenant une ou plusieurs bases d'oxydation, un ou plusieurs dérivés de 2,6-diamino toluène à titre de coupleur, et éventuellement un ou plusieurs coupleurs additionnels choisis parmi les coupleurs classiquement utilisés dans le domaine de la coloration d'oxydation, tels que la résorcine et ses dérivés, les dérivés naphtaléniques ou bien encore les dérivés pyridiniques. Cependant bien que les colorations obtenues en mettant en oeuvre de telles compositions soient très chromatiques, elles ne sont pas entièrement satisfaisantes, notamment du point de vue de leur ténacité vis à vis des différents traitements et agressions naturelles que peuvent subir les fibres kératiniques.

Or, la demanderesse vient maintenant de découvrir qu'il est possible d'obtenir de nouvelles teintures, capables de conduire à des colorations puissantes et très chromatiques, et résistant bien aux diverses agressions que peuvent subir les fibres, en associant au moins une base d'oxydation, du 1,3-bis-(β-hydroxyéthyl) amino 2-méthyl benzène et/ou au moins l'un de ses sels d'addition avec un acide à titre de premier coupleur, et au moins un coupleur hétérocyclique convenablement sélectionné à titre de second coupleur.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- du 1,3-bis-(β-hydroxyéthyl)amino 2-méthyl benzène et/ou au moins l'un de ses sels d'addition avec un acide à titre de premier coupleur ;
- et au moins un coupleur hétérocyclique selon la revendication 1 à titre de second coupleur ;

La composition tinctoriale conforme à l'invention conduit à des colorations puissantes, très chromatiques, et présentant d'excellentes propriétés de résistances à la fois vis à vis des agents atmosphériques tels que la lumière et les intempéries et vis à vis de la transpiration et des différents traitements que peuvent subir les cheveux.

L'invention a également pour objet un procédé de teinture d'oxydation des fibres kératiniques mettant en oeuvre cette composition tinctoriale.

Les coupleurs hétérocycliques utilisables à titre de second coupleur dans la composition tinctoriale sont les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

Parmi les dérivés indoliques utilisables à titre de coupleurs hétérocycliques dans la composition tinctoriale conforme à l'invention, on plus particulièrement citer les composés de formule (I) suivante, et leurs sels d'addition avec un acide : 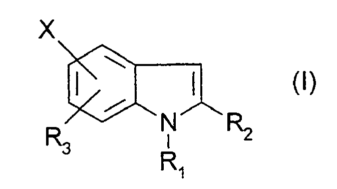 dans laquelle :
R₁ représente un atome d'hydrogène, un radical alkyle en C₁-C₄, un radical mono- ou polyhydroxyalkyle en C₂-C₄ ou aminoalkyle en C₁-C₄ dont l'amine est mono ou disubstituée par un groupement alkyle en C₁-C₄ ;
R₂ représente un atome d'hydrogène ou un radical alkyle en C₁-C₄ ;
R₃ représente un atome d'hydrogène, un radical alkyle en C₁-C₄ ou hydroxyle ;
X représente un radical hydroxyle ou NHR₄ dans lequel R₄ représente un atome d'hydrogène, un radical alkyle en C₁-C₄ ou hydroxyalkyle en C₁-C₄.

Parmi les dérivés indoliques de formule (I) ci-dessus, on peut plus particulièrement citer le 4-hydroxy indole, le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, le 4-hydroxy 1-N-(β-hydroxyéthyl) indole, le 4-hydroxy 1-N-(β-hydroxypropyl) indole, le 1-N-(β,γ-dihydroxypropyl) 4-hydroxy indole, le 4-hydroxy 1-N-(β-hydroxyéthyl) 5-méthyl indole, le 1-N-(γ-diméthylaminopropyl) 4-hydroxy indole, et leur sels d'addition avec un acide.

Parmi les dérivés indoliniques utilisables à titre de coupleurs hétérocycliques dans la composition tinctoriale conforme à l'invention, on peut particulièrement citer la 4-hydroxy indoline, la 6-hydroxy indoline, la 6-amino indoline, la 5,6-dihydroxy indoline, et leurs sels d'addition avec un acide.

Parmi les dérivés de benzimidazole utilisables à titre de coupleurs hétérocycliques dans la composition tinctoriale conforme à l'invention, on peut plus particulièrement citer les composés de formule (II) suivante, et leurs sels d'addition avec un acide : 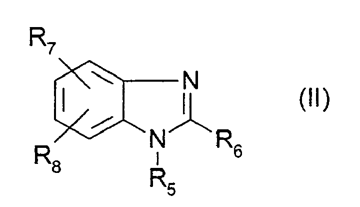 dans laquelle :
R₅ représente un atome d'hydrogène ou un radical alkyle en C₁-C₄,
R₆ représente un atome d'hydrogène, un radical alkyle en C₁-C₄ ou phényle,
R₇ représente un radical hydroxyle, amino ou méthoxy,
R₈ représente un atome d'hydrogène, un radical hydroxyle, méthoxy ou alkyle en C₁-C₄;
sous réserve que :
- lorsque R₇ désigne un radical amino, alors il occupe la position 4,
- lorsque R₇ occupe la position 4, alors R₈ occupe la position 7,
- lorsque R₇ occupe la position 5, alors R₈ occupe la position 6.

Parmi les dérivés de benzimidazole de formule (II) ci-dessus, on peut plus particulièrement citer le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

Parmi les dérivés de benzomorpholine utilisables à titre de coupleurs hétérocycliques dans la composition tinctoriale conforme à l'invention, on peut plus particulièrement citer les composés de formule (III) suivante, et leurs sels d'addition avec un acide : 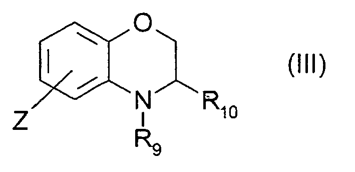 dans laquelle :
R₉ et R₁₀, identiques ou différents, représentent un atome d'hydrogène ou un radical alkyle en C₁-C₄,
Z représente un radical hydroxyle ou amino.

Parmi les dérivés de benzomorpholine de formule (III) ci-dessus, on peut plus particulièrement citer la 6-hydroxy 1,4-benzomorpholine, la N-méthyl 6-hydroxy 1,4-benzomorpholine, la 6-amino 1,4-benzomorpholine, et leurs sels d'addition avec un acide.

Parmi les dérivés de sésamol utilisables à titre de coupleur hétérocyclique dans la composition tinctoriale conforme à l'invention, on peut particulièrement citer les composés de formule (IV) suivante, et leurs sels d'addition avec un acide : 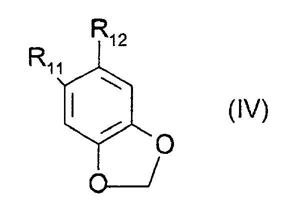 dans laquelle :
R₁₁ désigne un radical hydroxyle, amino, alkyl(C₁-C₄)amino, monohydroxyalkyl(C₁-C₄)amino ou polyhydroxyalkyl(C₂-C₄)amino,
R₁₂ désigne un atome d'hydrogène ou d'halogène ou un radical alcoxy en C₁-C₄.

Parmi les dérivés de sésamol de formule (IV) ci-dessus, on peut plus particulièrement citer le 2-bromo 4,5-méthylènedioxy phénol, la 2-méthoxy 4,5-méthylènedioxy aniline, le 2-(β-hydroxyéthyl)amino 4,5-méthylènedioxy benzène, et leurs sels d'addition avec un acide.

Parmi les dérivés pyrazolo-azoliques utilisables à titre de coupleur hétérocyclique dans la composition tinctoriale conforme à l'invention, on peut plus particulièrement citer les composés décrits dans les demandes de brevets et brevets suivants : FR 2 075 583, EP-A-119 860, EP-A-285 274, EP-A-244 160, EP-A-578 248, GB 1 458 377, US 3 227 554, US 3 419 391, US 3 061 432, US 4 500 630, US 3 725 067, US 3 926 631, US 5 457 210, JP 84/99437, JP 83/42045, JP 84/162548, JP 84/171956, JP 85/33552, JP 85/43659, JP 85/172982, JP 85/190779 ainsi que dans les publications suivantes : Chem. Ber. 32, 797 (1899), Chem. Ber. 89, 2550, (1956), J. Chem. Soc. Perkin trans I, 2047, (1977), J. Prakt. Chem., 320, 533, (1978).

A titre de dérivés pyrazolo-azoliques, on peut tout particulièrement citer :
- le 2-méthyl pyrazolo [1,5-b]-1,2,4-triazole,
- le 2-éthyl pyrazolo [1,5-b]-1,2,4-triazole,
- le 2-isopropyl pyrazolo [1,5-b]-1,2,4-triazole,
- le 2-phényl pyrazolo [1,5-b]-1,2,4-triazole,
- le 2,6-diméthyl pyrazolo [1,5-b]- 1,2,4-triazole ,
- le 7-chloro-2,6-diméthylpyrazolo[1,5-b]-1,2,4-triazole,
- le 3,6-diméthyl-pyrazolo [3,2-c]-1,2,4-triazole,
- le 6-phényl-3-méthylthio- pyrazolo [3,2-c]-1,2,4-triazole,
- le 6-amino- pyrazolo [1,5-a] benzimidazole,
et leurs sels d'addition avec un acide.

Parmi les dérivés pyrrolo-azoliques utilisables à titre de coupleur hétérocyclique dans la composition tinctoriale conforme à l'invention, on peut plus particulièrement citer les composés décrits dans les demandes de brevets et brevets suivants : US 5 256 526, EP-A-557 851, EP-A-578 248, EP-A-518 238, EP-A-456 226, EP-A-488 909, EP-A-488 248, et dans les publications suivantes :
- D.R. Liljegren Ber. 1964, 3436 ;
- E.J. Browne, J.C.S., 1962, 5149 ;
- P. Magnus, J.A.C.S., 1990, 112, 2465 ;
- P. Magnus, J.A.C.S., 1987, 109, 2711 ;
- Angew. Chem. 1960, 72, 956 ;
- et Rec. Trav. Chim. 1961, 80, 1075.

A titre de dérivés pyrrolo-azoliques, on peut tout particulièrement citer :
- le 5-cyano-4-éthoxycarbonyl-8-méthyl pyrrolo [1,2-b]-1,2,4-triazole,
- le 5-cyano-8-méthyl-4-phényl pyrrolo [1,2-b]-1,2,4-triazole,
- le 7-amido-6-éthoxycarbonyl pyrrolo [1,2-a]- benzimidazole, et leurs sels d'addition avec un acide.

Parmi les dérivés imidazolo-azoliques utilisables à titre de coupleur hétérocyclique dans la composition tinctoriale conforme à l'invention, on peut plus particulièrement citer les composés décrits dans les demandes de brevets et brevets suivants : US 5,441,863 ; JP 62-279 337 ; JP 06-236 011 et JP 07-092 632.

A titre de dérivés imidazolo-azoliques, on peut tout particulièrement citer :
- le 7,8-dicyano-imidazolo- [3,2-a]- imidazole,
- le 7,8-dicyano-4-méthyl-imidazolo- [3,2-a]- imidazole, et leurs sels d'addition avec un acide.

Parmi les dérivés pyrazolo-pyrimidiniques utilisables à titre de coupleur hétérocyclique dans la composition tinctoriale conforme à l'invention, on peut plus particulièrement citer les composés décrits dans la demande de brevet suivante : EP-A-304001.

A titre de dérivés pyrazolo-pyrimidiniques, on peut tout particulièrement citer :
- le pyrazolo [1,5-a] pyrimidin-7-one,
- le 2,5-diméthyl pyrazolo [1,5-a] pyrimidin-7-one,
- le 2-méthyl-6-éthoxycarbonyl pyrazolo [1,5-a] pyrimidin-7-one,
- le 2-méthyl-5-méthoxyméthyl pyrazolo [1,5-a] pyrimidin-7-one,
- le 2-ter-butyl-5-trifluorométhyl pyrazolo [1,5-a] pyrimidin-7-one,
- 2,7-diméthyl pyrazolo [1,5-a] pyrimidin-5-one,
et leurs sels d'addition avec un acide.

Parmi les dérivés de pyrazolin-3,5-diones utilisables à titre de coupleur hétérocyclique dans la composition tinctoriale conforme à l'invention, on peut plus particulièrement citer les composés décrits dans les demandes de brevets et brevets suivants : JP 07-036159, JP 07-084348 et US 4 128 425, et dans les publications suivantes :
- L. WYZGOWSKA, Acta. Pol. Pharm. 1982, 39 (1-3), 83.
- E. HANNIG, Pharmazie, 1980, 35 (4), 231
- M. H. ELNAGDI, Bull. Chem. Soc. Jap., 46 (6), 1830, 1973
- G. CARDILLO, Gazz. Chim. Ital. 1966, 96, (8-9), 973.

A titre de dérivés de pyrazolin-3,5-diones, on peut tout particulièrement citer :
- la 1,2-diphényl pyrazolin-3,5-dione,
- la 1,2-diéthyl pyrazolin-3,5-dione,
et leurs sels d'addition avec un acide.

Parmi les dérivés pyrrolo-[3,2-d]-oxazoliques utilisables à titre de coupleur hétérocyclique dans la composition tinctoriale conforme à l'invention, on peut plus particulièrement citer les composés décrits dans la demande de brevet JP 07 325 375.

Parmi les dérivés pyrazolo-[3,4-d]-thiazoliques utilisables à titre de coupleur hétérocyclique dans la composition tinctoriale conforme à l'invention, on peut plus particulièrement citer les composés décrits dans la demandes de brevet JP 07 244 361 et dans J. Heterocycl. Chem. 16, 13, (1979).

Parmi les dérivés S-oxyde-thiazolo-azoliques et S,S-dioxyde-thiazolo-azoliques utilisables à titre de coupleur hétérocyclique dans la composition tinctoriale conforme à l'invention, on peut plus particulièrement citer les composés décrits dans les documents suivants :
- JP 07 09 84 89 ;
- Khim. Geterotsilk. Soedin, 1967, p. 93 ;
- J. Prakt. Chem., 318, 1976, p. 12 ;
- Indian J. Heterocycl. Chem. 1995, 5 (2), p. 135 ;
- Acta. Pol. Pharm. 1995, 52 (5), 415 ;
- Heterocycl. Commun. 1995, 1 (4), 297 ;
- Arch. Pharm. (Weinheim, Ger.), 1994, 327 (12), 825.

La nature de la ou des bases d'oxydation utilisées dans la composition tinctoriale conforme à l'invention n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (VI) suivante et leurs sels d'addition avec.un acide : 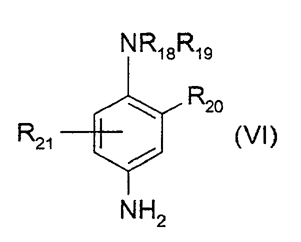 dans laquelle :
- R₁₈ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₁₉ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄)ou alkyle en C₁-C₄ substitué par un groupement azoté ;
- R₂₀ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₂₁ représente un atome d'hydrogène, d'halogène ou un radical alkyle en C₁-C₄.

Parmi les groupements azotés de la formule (VI) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (VI) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (VI) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (VII) suivante, et leurs sels d'addition avec un acide : 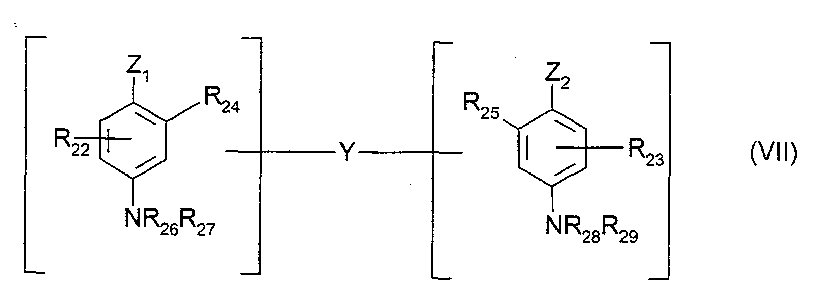 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₂₂ et R₂₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₂₄, R₂₅, R₂₆, R₂₇, R₂₈ et R₂₉, identiques ou différents, représentent un atome d'hydrogène, un bras de liaison Y ou un radical alkyle en C₁-C₄ ;
   étant entendu que les composés de formule (VII) ne comportent qu'un seul bras de liaison Y par molécule.

Parmi les groupements azotés de la formule (VII) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (VII) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (VII), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés de formule (VIII) suivante, et leurs sels d'addition avec un acide : 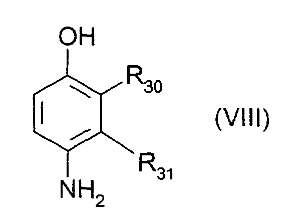 dans laquelle :
- R₃₀ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄
ou hydroxyalkyl(C₁-C₄)aminoalkyle en C₁-C₄,
- R₃₁ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
   étant entendu qu'au moins un des radicaux R₃₀ et R₃₁ représente un atome d'hydrogène.

Parmi les para-aminophénols de formule (VIII) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

Le 1,3-bis-(β-hydroxyéthyl)amino 2-méthyl benzène et/ou le ou ses sels d'addition avec un acide utilisés à titre de premier coupleur selon l'invention, représentent de préférence de 0,001 à 10 % en poids environ du poids total de la composition tinctoriale et encore plus préférentiellement de 0,01 à 5 % en poids environ de ce poids.

Le ou les coupleurs hétérocycliques représentent de préférence de 0,0001 à 10 % en poids environ du poids total de la composition tinctoriale conforme à l'invention et encore plus préférentiellement de 0,005 à 5 % en poids environ de ce poids.

La composition tinctoriale conforme à l'invention peut en outre contenir un ou plusieurs coupleurs additionnels différents du 1,3-bis-(β-hydroxyéthyl)amino 2-méthyl benzène, des coupleurs hétérocycliques conformes à l'invention et de leurs sels d'addition avec un acide et/ou un ou plusieurs colorants directs, notamment pour modifier les nuances ou les enrichir en reflets.

Parmi les coupleurs pouvant être présents à titre additionnel dans la composition tinctoriale conforme à l'invention, on peut notamment citer les coupleurs benzéniques tels que par exemple les méta-aminophénols, les métaphénylènediamines, les métadiphénols, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IX) suivante: 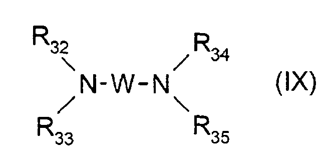 dans laquelle W est un reste propylène éventuellement substitué par un groupement hydroxyle ou un radical alkyle en C₁-C₄ ; R₃₂, R₃₃, R₃₄ et R₃₅, identiques ou différents, représentent un atome d'hydrogène, un radical alkyle en C₁-C₄ ou hydroxyalkyle en C₁-C₄.

La composition tinctoriale conforme à l'invention peut également renfermer divers adjuvants utilisés classiquement dans les compositions pour la teinture des cheveux.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les oxydo-réductases à 2 électrons, les peroxydases et les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES

### EXEMPLES 1 à 3 DE TEINTURE EN MILIEU ALCALIN

On a préparé les compositions tinctoriales conformes à l'invention suivantes (teneurs en grammes) :

| **EXEMPLE** | **1** | **2** | **3** |
|---|---|---|---|
| Paraphénylènediamine (base d'oxydation) | 0,22 | - | - |
| Para-aminophénol (base d'oxydation) | - | 0,44 | - |
| Pyrazolo-[1,5-a]-pyrimidine-3,7-diamine, 2HCl (base d'oxydation) | - | - | 0,88 |
| 1,3-bis-(β-hydroxyéthyl)amino 2-méthyl benzène (premier coupleur) | 0,21 | 0,21 | 0,21 |
| 3,6-diméthyl-pyrazolo [3,2-c]-1,2,4-triazole (second coupleur) | 0,14 | - | - |
| 4-hydroxy indole (second coupleur) | - | 0,26 | - |
| 6-hydroxy 1,4-benzomorpholine (second coupleur) | - | - | 0,30 |
| Support de teinture commun | (*) | (*) | (*) |
| Eau déminéralisée q.s.p. | 100 g | 100 g | 100 g |

(*) : Support de teinture commun n°1 :
- Ethanol à 96° 18 g
- Métabisulfite de sodium en solution aqueuse à 35% 0,68 g
- Sel pentasodique de l'acide diéthylène triamino pentacétique 1,1 g
- Ammoniaque à 20% de NH₃ 10 g

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.

Les cheveux ont été teints dans les nuances figurent dans le tableau ci-après :

| **EXEMPLE** | **NUANCE OBTENUE** |
|---|---|
| **1** | Violine irisé |
| **2** | Rouge acajou |
| **3** | Irisé cendré |

### EXEMPLE 4 DE TEINTURE EN MILIEU ACIDE

On a préparé la composition tinctoriale non conforme à l'invention suivante :
- Sulfate de N,N-bis-(β-hydroxyéthyl) paraphénylènediamine (base d'oxydation) 0,63 g
- 1,3-bis-(β-hydroxyéthyl)amino 2-méthyl benzène (premier coupleur) 0,21 g
- 2,6-dihydroxy 3,4-diméthyl pyridine (second coupleur) 0,14 g
- Ethanol à 96° 18 g
- Métabisulfite de sodium en solution aqueuse à 35% 0,68 g
- Sel pentasodique de l'acide diéthylène triamino pentacétique 1,1 g
- Tampon K₂HPO₄ / KH₂PO₄ (1,5 M / 1 M) 10 g
- Eau déminéralisée qsp 100 g

Au moment de l'emploi, on a mélangé la composition tinctoriale décrites ci-dessus avec une quantité pondérale équivalente de peroxyde d'hydrogène à 20 volumes (6% en poids) présentant un pH d'environ 3.

Le mélange résultant présentait un pH d'environ 6,8 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.

Les cheveux ont été teints dans une nuance violacé cendré.

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :
- au moins une base d'oxydation,
- du 1,3-bis-(β-hydroxyéthyl)amino 2-méthyl benzène et/ou au moins l'un de ses sels d'addition avec un acide à titre de premier coupleur ;
- et au moins un coupleur hétérocyclique à titre de second coupleur choisis parmi les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

2. Composition selon la revendication 1, **caractérisée par le fait que** le ou les dérivés indoliques sont choisis parmi les composés de formule (I) suivante, et leurs.sels d'addition avec un acide : 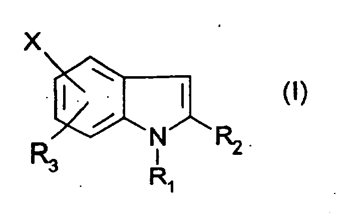 dans laquelle :
R₁ représente un atome d'hydrogène, un radical alkyle en C₁-C₄, un radical mono- ou polyhydroxyalkyle en C₂-C₄ ou aminoalkyle en C₁-C₄ dont l'amine est mono ou disubstituée par un groupement alkyle en C₁-C₄ ;
R₂ représente un atome d'hydrogène ou un radical alkyle en C₁-C₄ ;
R₃ représente un atome d'hydrogène, un radical alkyle en C₁-C₄ ou hydroxyle ;
X représente un radical hydroxyle ou NHR₄ dans lequel R₄ représente un atome d'hydrogène, un radical alkyle en C₁-C₄ ou hydroxyalkyle en C₁-C₄.

3. Composition selon la revendication 2, **caractérisée par le fait que** les dérivés indoliques de formule (I) sont choisis parmi le 4-hydroxy indole, le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, le 4-hydroxy 1-N-(β-hydroxyéthyl) indole, le 4-hydroxy 1-N-(β-hydroxypropyl) indole, le 1-N-(β,γ-dihydroxypropyl) 4-hydroxy indole, le 4-hydroxy 1-N-(β-hydroxyéthyl) 5-méthyl indole, le 1-N-(γ-diméthylaminopropyl) 4-hydroxy indole, et leur sels d'addition avec un acide.

4. Composition selon la revendication 3, **caractérisée par le fait que** les dérivés indoliniques sont choisis parmi la 4-hydroxy indoline, la 6-hydroxy indoline, la 6-amino indoline, la 5,6-dihydroxy indoline, et leurs sels d'addition avec un acide.

5. Composition selon la revendication 1, **caractérisée par le fait que** les dérivés de benzimidazole sont choisis parmi les composés de formule (II) suivante, et leurs sels d'addition avec un acide : 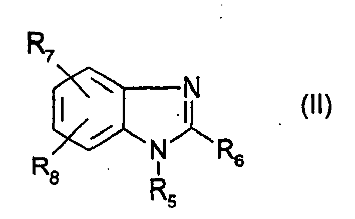 dans laquelle :
R₅ représente un atome d'hydrogène ou un radical alkyle en C₁-C₄,
R₆ représente un atome d'hydrogène, un radical alkyle en C₁-C₄ ou phényle,
R₇ représente un radical hydroxyle, amino ou méthoxy,
R₈ représente un atome d'hydrogène, un radical hydroxyle, méthoxy ou alkyle en C₁-C₄ ;
sous réserve que :
- lorsque R₇ désigne un radical amino, alors il occupe la position 4,
- lorsque R₇ occupe la position 4, alors R₈ occupe la position 7,
- lorsque R₇ occupe la position 5, alors R₈ occupe la position 6.

6. Composition selon la revendication 5, **caractérisée par le fait que** les dérivés de benzimidazole de formule (II) sont choisis parmi le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

7. Composition selon la revendication 1, **caractérisée par le fait que** les dérivés de benzomorpholine sont choisis parmi les composés de formule (III) suivante, et leurs sels d'addition avec un acide : 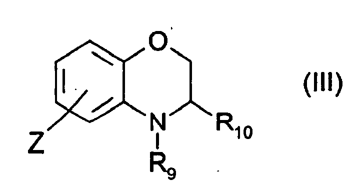 dans laquelle :
R₉ et R₁₀, identiques ou différents, représentent un atome d'hydrogène ou un radical alkyle en C₁-C₄,
Z représente un radical hydroxyle ou amino.

8. Composition selon la revendication 7, **caractérisée par le fait que** les dérivés de benzomorpholine de formule (III) sont choisis parmi la 6-hydroxy 1,4-benzomorpholine, la N-méthyl 6-hydroxy 1,4-benzomorpholine, la 6-amino 1,4-benzomorpholine, et leurs sels d'addition avec un acide.

9. Composition selon la revendication 1, **caractérisée par le fait que** les dérivés de sésamol sont choisis parmi les composés de formule (IV) suivante, et leurs sels d'addition avec un acide : 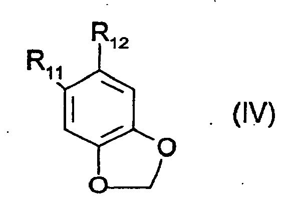 dans laquelle:
R₁₁ désigne un radical hydroxyle, amino, alkyl(C₁-C₄)amino, monohydroxyalkyl(C₁-C₄)amino ou polyhydroxyalkyl(C₂-C₄)amino,
R₁₂ désigne un atome d'hydrogène ou d'halogène ou un radical alcoxy en C₁-C₄.

10. Composition selon la revendication 9, **caractérisée par le fait que** les dérivés de sésamol de formule (IV) sont choisis parmi le 2-bromo 4,5-méthylènedioxy phénol, la 2-méthoxy 4,5-méthylènedioxy aniline, le 2-(β-hydroxyéthyl)amino 4,5-méthylènedioxy benzène, et leurs sels d'addition avec un acide.

11. Composition selon la revendication 1, **caractérisée par le fait que** les dérivés pyrazolo-azoliques sont choisis parmi le 2-méthyl pyrazolo [1,5-b]-1,2,4-triazole, le 2-éthyl pyrazolo [1,5-b]-1,2,4-triazole, le 2-isopropyl pyrazolo [1,5-b]-1,2,4-triazole, le 2-phényl pyrazolo [1,5-b]-1,2,4-triazole, le 2,6-diméthyl pyrazolo [1,5-b]-1,2,4-triazole, le 7-chloro-2,6-diméthylpyrazolo[1,5-b]-1,2,4-triazole, le 3,6-diméthyl-pyrazolo [3,2-c]-1,2,4-triazole, le 6-phényl-3-méthylthio- pyrazolo [3,2-c]-1,2,4-triazole, le 6-amino- pyrazolo [1,5-a] benzimidazole, et leurs sels d'addition avec un acide.

12. Composition selon la revendication 1, **caractérisée par le fait que** les dérivés pyrrolo-azoliques sont choisis parmi le 5-cyano-4-éthoxycarbonyl-8-méthyl pyrrolo [1,2-b]-1,2,4-triazole, le 5-cyano-8-méthyl-4-phényl pyrrolo [1,2-b]-1,2,4-triazole, le 7-amido-6-éthoxycarbonyl pyrrolo [1,2-a]- benzimidazole, et leurs sels d'addition avec un acide.

13. Composition selon la revendication 1, **caractérisée par le fait que** les dérivés imidazolo-azoliques sont choisis parmi le 7,8-dicyano-imidazolo [3,2-a]-imidazole, le 7,8-dicyano-4-méthyl-imidazolo- [3,2-a]- imidazole, et leurs sels d'addition avec un acide.

14. Composition selon la revendication 1, **caractérisée par le fait que** les dérivés pyrazolo-pyrimidiniques sont choisis parmi - le pyrazolo [1,5-a] pyrimidin-7-one, le 2,5-diméthyl pyrazolo [1,5-a] pyrimidin-7-one, le 2-méthyl-6-éthoxycarbonyl pyrazolo [1,5-a] pyrimidin-7-one, le 2-méthyl-5-méthoxyméthyl pyrazolo [1,5-a] pyrimidin-7-one, le 2-ter-butyl-5-trifluorométhyl pyrazolo [1,5-a] pyrimidin-7-one, 2,7-diméthyl pyrazolo [1,5-a] pyrimidin-5-one, et leurs sels d'addition avec un acide.

15. Composition selon la revendication 1, **caractérisée par le fait que** les dérivés de pyrazolin-3,5-diones sont choisis parmi la 1,2-diphényl pyrazolin-3,5-dione, la 1,2-diéthyl pyrazolin-3,5-dione, et leurs sels d'addition avec un acide.

16. Composition selon l'une quelconque des revendications précédentes, **caractérisée par le fait que** la ou les bases d'oxydation sont choisies parmi les paraphénylènediamines, les bases doubles, les para-aminophénols, les ortho aminophénols et les bases d'oxydation hétérocycliques.

17. Composition selon l'une quelconque des revendications précédentes, **caractérisée par le fait que** la ou les bases d'oxydation représentent de 0,0005 à 12 % en poids du poids total de la composition tinctoriale.

18. Composition selon la revendication 17, **caractérisée par le fait que** la ou les bases d'oxydation représentent de 0,005 à 6 % en poids du poids total de la composition tinctoriale.

19. Composition selon l'une quelconque des revendications précédentes, **caractérisée par le fait que** le 1,3-bis-(β-hydroxyéthyl)amino 2-méthyl benzène et/ou le ou ses sels d'addition avec un acide représentent de 0,001 à 10 % en poids du poids total de la composition tinctoriale.

20. Composition selon la revendication 19, **caractérisée par le fait que** le 1,3-bis-(β-hydroxyéthyl)amino 2-méthyl benzène et/ou le ou ses sels d'addition avec un acide représentent de 0,01 à 5 % en poids du poids total de la composition tinctoriale.

21. Composition selon l'une quelconque des revendications précédentes, **caractérisée par le fait que** le ou les coupleurs hétérocycliques représentent de 0,0001 à 10 % en poids du poids total de la composition tinctoriale.

22. Composition selon la revendication 21, **caractérisée par le fait que** le ou les coupleurs hétérocycliques représentent de 0,005 à 5 % en poids du poids total de la composition tinctoriale.

23. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

24. Procédé de teinture des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 23, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

25. Procédé selon la revendication 24, **caractérisé par le fait que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides, et les enzymes.

26. Dispositif à plusieurs compartiments, ou "kit" de teinture à plusieurs compartiments, dont un premier compartiment renferme une composition tinctoriale telle que définie à l'une quelconque des revendications 1 à 23 et un second compartiment renferme une composition oxydante.

## Claims

1. Composition for the oxidation dyeing of keratin fibres, and in particular human keratin fibres such as the hair, **characterized in that** it comprises, in a medium which is suitable for dyeing:
- at least one oxidation base,
- 1,3-bis(β-hydroxyethyl)amino-2-methylbenzene and/or at least one of the addition salts thereof with an acid, as first coupler; and
- at least one heterocyclic coupler, as second coupler chosen from indole derivatives, indoline derivatives, benzimidazole derivatives, benzomorpholine derivatives, sesamol derivatives, pyrazoloazole derivatives, pyrroloazole derivatives, imidazoloazole derivatives, pyrazolopyrimidine derivatives, pyrazoline-3,5-dione derivatives, pyrrolo[3,2-d]oxazole derivatives, pyrazolo[3,4-d]thiazole derivatives, thiazoloazole S-oxide derivatives and thiazoloazole S,S-dioxide derivatives, and the addition salts thereof with an acid.

2. Composition according to Claim 1, **characterized in that** the indole derivative(s) is (are) chosen from the compounds of formula (I) below, and the addition salts thereof with an acid: 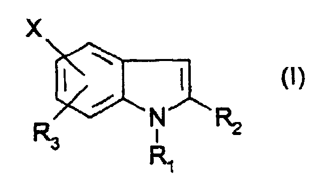 in which:
R₁ represents a hydrogen atom, a C₁-C₄ alkyl radical, a C₂-C₄ mono- or polyhydroxyalkyl radical or a C₁-C₄ aminoalkyl radical in which the amine is mono- or disubstituted with a C₁-C₄ alkyl group;
R₂ represents a hydrogen atom or a C₁-C₄ alkyl radical;
R₃ represents a hydrogen atom or a C₁-C₄ alkyl or hydroxyl radical;
X represents a hydroxyl radical or a radical NHR₄ in which R₄ represents a hydrogen atom or a C₁-C₄ alkyl or C₁-C₄ hydroxyalkyl radical.

3. Composition according to Claim 2, **characterized in that** the indole derivatives of formula (I) are chosen from 4-hydroxyindole, 6-hydroxyindole, 7-aminoindole, 6-aminoindole, 7-hydroxyindole, 7-ethyl-6-(β-hydroxyethyl)aminoindole, 4-aminoindole, 6-hydroxy-1-methylindole, 5,6-dihydroxyindole, 4-hydroxy-1-N-methylindole, 4-hydroxy-2-methylindole, 4-hydroxy-5-methylindole, 4-hydroxy-l-N-(β-hydroxyethyl)indole, 4-hydroxy-1-N-(β-hydroxypropyl)indole, 1-N-(β,γ-dihydroxypropyl)-4-hydroxyindole, 4-hydroxy-1-N-(β-hydroxyethyl)-5-methylindole and 1-N-(γ-dimethylaminopropyl)-4-hydroxyindole, and the addition salts thereof with an acid.

4. Composition according to Claim 3, **characterized in that** the indoline derivatives are chosen from 4-hydroxyindoline, 6-hydroxyindoline, 6-aminoindoline and 5,6-dihydroxyindoline, and the addition salts thereof with an acid.

5. Composition according to Claim 1, **characterized in that** the benzimidazole derivatives are chosen from the compounds of formula (II) below, and the addition salts thereof with an acid: 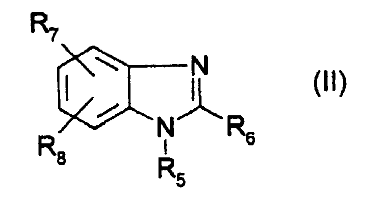 in which:
R₅ represents a hydrogen atom or a C₁-C₄ alkyl radical,
R₆ represents a hydrogen atom or a C₁-C₄ alkyl or phenyl radical,
R₇ represents a hydroxyl, amino or methoxy radical,
R₈ represents a hydrogen atom or a hydroxyl, methoxy or C₁-C₄ alkyl radical;
with the proviso that:
- when R₇ denotes an amino radical, then it occupies position 4,
- when R₇ occupies position 4, then R₈ occupies position 7,
- when R₇ occupies position 5, then R₈ occupies position 6.

6. Composition according to Claim 5, **characterized in that** the benzimidazole derivatives of formula (II) are chosen from 4-hydroxybenzimidazole, 4-aminobenzimidazole, 4-hydroxy-7-methylbenzimidazole, 4-hydroxy-2-methylbenzimidazole, 1-butyl-4-hydroxybenzimidazole, 4-amino-2-methylbenzimidazole, 5,6-dihydroxybenzimidazole, 5-hydroxy-6-methoxybenzimidazole, 4,7-dihydroxybenzimidazole, 4,7-dihydroxy-1-methylbenzimidazole, 4,7-dimethoxybenzimidazole, 5,6-dihydroxy-1-methylbenzimidazole, 5,6-dihydroxy-2-methylbenzimidazole and 5,6-dimethoxybenzimidazole, and the addition salts thereof with an acid.

7. Composition according to Claim 1, **characterized in that** the benzomorpholine derivatives are chosen from the compounds of formula (III) below, and the addition salts thereof with an acid: 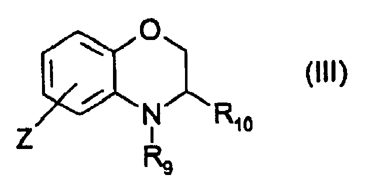 in which:
R₉ and R₁₀, which may be identical or different, represent a hydrogen atom or a C₁-C₄ alkyl radical,
Z represents a hydroxyl or amino radical.

8. Composition according to Claim 7, **characterized in that** the benzomorpholine derivatives of formula (III) are chosen from 6-hydroxy-1,4-benzomorpholine, N-methyl-6-hydroxy-1,4-benzomorpholine and 6-amino-1,4-benzomorpholine, and the addition salts thereof with an acid.

9. Composition according to Claim 1, **characterized in that** the sesamol derivatives are chosen from the compounds of formula (IV) below, and the addition salts thereof with an acid: 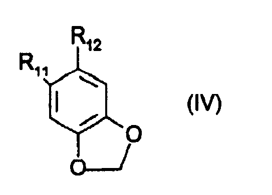 in which:
R₁₁ denotes a hydroxyl, amino, (C₁-C₄) alkylamino, monohydroxy(C₁-C₄)alkylamino or polyhydroxy(C₂-C₄)alkylamino radical,
R₁₂ denotes a hydrogen or halogen atom or a C₁-C₄ alkoxy radical.

10. Composition according to Claim 9, **characterized in that** the sesamol derivatives of formula (IV) are chosen from 2-bromo-4,5-methylenedioxyphenol, 2-methoxy-4,5-methylenedioxyaniline and 2-(β-hydroxyethyl)amino-4,5-methylenedioxybenzene, and the addition salts thereof with an acid.

11. Composition according to Claim 1, **characterized in that** the pyrazoloazole derivatives are chosen from 2-methylpyrazolo[1,5-b]-1,2,4-triazole, 2-ethylpyrazolo-[1,5-b]-1,2,4-triazole, 2-isopropylpyrazolo[1,5-b]-1,2,4-triazole, 2-phenylpyrazolo[1,5-b]-1,2,4-triazole, 2,6-dimethylpyrazolo[1,5-b]-1,2,4-triazole, 7-chloro-2,6-dimethylpyrazolo[1,5-b]-1,2,4-triazole, 3,6-dimethylpyrazolo[3,2-c]-1,2,4-triazole, 6-phenyl-3-methylthiopyrazolo[3,2-c]-1,2,4-triazole and 6-aminopyrazolo-[1,5-a]benzimidazole, and the addition salts thereof with an acid.

12. Composition according to Claim 1, **characterized in that** the pyrroloazole derivatives are chosen from 5-cyano-4-ethoxycarbonyl-8-methylpyrrolo[1,2-b]-1,2,4-triazole, 5-cyano-8-methyl-4-phenylpyrrolo[1,2-b]-1,2,4-triazole, 7-amido-6-ethoxycarbonylpyrrolo[1,2-a]benzimidazole, and the addition salts thereof with an acid.

13. Composition according to Claim 1, **characterized in that** the imidazoloazole derivatives are chosen from 7,8-dicyanoimidazolo[3,2-a]imidazole and 7,8-dicyano-4-methylimidazolo[3,2-a]imidazole, and the addition salts thereof with an acid.

14. Composition according to Claim 1, **characterized in that** the pyrazolopyrimidine derivatives are chosen from pyrazolo[1,5-a]pyrimidin-7-one, 2,5-dimethylpyrazolo-[1,5-a]pyrimidin-7-one, 2-methyl-6-ethoxycarbonylpyrazolo[1,5-a]pyrimidin-7-one, 2-methyl-5-methoxymethylpyrazolo[1,5-a]pyrimidin-7-one, 2-tert-butyl-5-trifluoromethylpyrazolo[1,5-a]pyrimidin-7-one, 2,7-dimethylpyrazolo[1,5-a]pyrimidin-5-one, and the addition salts thereof with an acid.

15. Composition according to Claim 1, **characterized in that** the pyrazoline-3,5-dione derivatives are chosen from 1,2-diphenylpyrazoline-3,5-dione and 1,2-diethylpyrazoline-3,5-dione, and the addition salts thereof with an acid.

16. Composition according to any one of the preceding claims, **characterized in that** the oxidation base(s) is (are) chosen from para-phenylenediamines, double bases, para-aminophenols, ortho-aminophenols and heterocyclic oxidation bases.

17. Composition according to any one of the preceding claims, **characterized in that** the oxidation base(s) represent(s) from 0.0005 to 12% by weight relative to the total weight of the dye composition.

18. Composition according to Claim 17, **characterized in that** the oxidation base(s) represent(s) from 0.005 to 6% by weight relative to the total weight of the dye composition.

19. Composition according to any one of the preceding claims, **characterized in that** the 1,3-bis(β-hydroxyethyl)amino-2-methylbenzene and/or the addition salt(s) thereof with an acid represent(s) from 0.001 to 10% by weight relative to the total weight of the dye composition.

20. Composition according to Claim 19, **characterized in that** the 1,3-bis(β-hydroxyethyl)amino-2-methylbenzene and/or the addition salt(s) thereof with an acid represent(s) from 0.01 to 5% by weight relative to the total weight of the dye composition.

21. Composition according to any one of the preceding claims, **characterized in that** the heterocyclic coupler(s) represent(s) from 0.0001 to 10% by weight relative to the total weight of the dye composition.

22. Composition according to Claim 21, **characterized in that** the heterocyclic coupler(s) represent(s) from 0.005 to 5% by weight relative to the total weight of the dye composition.

23. Composition according to any one of the preceding claims, **characterized in that** the addition salts with an acid are chosen from the hydrochlorides, hydrobromides, sulphates, tartrates, lactates and acetates.

24. Process for dyeing keratin fibres, and in particular human keratin fibres such as the hair, **characterized in that** at least one dye composition as defined in any one of Claims 1 to 23 is applied to the said fibres and **in that** the colour is developed at acidic, neutral or alkaline pH using an oxidizing agent which is added to the dye composition just at the time of use, or which is present in an oxidizing composition that is applied simultaneously or sequentially.

25. Process according to Claim 26, **characterized in that** the oxidizing agent present in the oxidizing composition is chosen from hydrogen peroxide, urea peroxide, alkali metal bromates, persalts such as perborates, percarbonates and persulphates, peracids and enzymes.

26. Multi-compartment dyeing device or multi-compartment dyeing "kit", a first compartment of which contains a dye composition as defined in any one of Claims 1 to 23, and a second compartment of which contains an oxidizing composition.

## Patentansprüche

1. Zusammensetzung zum oxidativen Färben von Keratinfasern und insbesondere menschlichen Keratinfasern wie Haaren, **dadurch gekennzeichnet, dass** sie in einem zum Färben geeigneten Medium enthält:
- mindestens eine Oxidationsbase,
- 1,3-Bis(β-hydroxyethyl)amino-2-methylbenzol und/oder mindestens eines seiner Additionssalze mit einer Säure als ersten Kuppler;
- und mindestens einen heterocyclischen Kuppler als zweiten Kuppler, der ausgewählt ist unter den Indolderivaten, Indolinderivaten, Benzimidazolderivaten, Benzomorpholinderivaten, Sesamolderivaten, Pyrazolo-azolderivaten, Pyrroloazolderivaten, Imidazolo-azolderivaten, Pyrazolo-pyrimidinderivaten, Pyrazolin-3,5-dionderivaten, Pyrrolo-[3,2-d]-oxazolderivaten, Pyrazolo-[3,4-d]-thiazolderivaten, S-Oxidthiazolo-azolderivaten, S,S-Dioxid-thiazolo-azolderivaten und ihren Additionssalzen mit einer Säure.

2. Zusammensetzung nach Anspruch 1, **dadurch gekennzeichnet, dass** die Indolverbindung oder die Indolverbindungen unter den Verbindungen der folgenden Formel (I) und ihren Additionssalzen mit einer Säure ausgewählt sind: 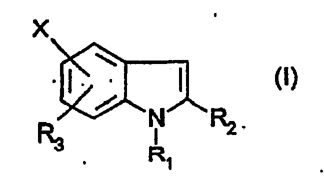 worin bedeuten:
R₁ ein Wasserstoffatom, eine Alkyl(C₁₋₄)gruppe, eine Mono- oder Polyhydroxyalkyl(C₂₋₄)gruppe oder eine Aminoalkyl(C₁₋₄)gruppe, deren Aminogruppe mit einer Alkyl(C₁₋₄)gruppe mono- oder disubstituiert ist;
R₂ ein Wasserstoffatom oder eine Alkyl(C₁₋₄)gruppe;
R₃ ein Wasserstoffatom, eine Alkyl(C₁₋₄)gruppe oder eine Hydroxygruppe;
X eine Hydroxygruppe oder NHR₄, wobei R₄ ein Wasserstoffatom, eine Alkyl(C₁₋₄)gruppe oder eine Hydroxyalkyl(C₁₋₄)gruppe bedeutet.

3. Zusammensetzung nach Anspruch 2, **dadurch gekennzeichnet, dass** die Indolderivate der Formel (I) unter 4-Hydroxyindol, 6-Hydroxyindol, 7-Aminoindol, 6-Aminoindol, 7-Hydroxyindol, 7-Ethyl-6-(β-hydroxyethyl)amino-indol, 4-Aminoindol, 6-Hydroxy-1-methylindol, 5,6-Dihydroxyindol, 4-Hydroxy-1-N-methylindol, 4-Hydroxy-2-methylindol, 4-Hydroxy-5-methylindol, 4-Hydroxy-1-N-(β-hydroxyethyl)-indol, 4-Hydroxy-1-N-(β-hydroxypropyl)-indol, 1-N-(β,γ-Dihydroxypropyl)-4-hydroxyindol, 4-Hydroxy-1-N-(β-hydroxyethyl)-5-methylindol, 1-N-(y-Dimethylaminopropyl)-4-hydroxyindol und ihren Additionssalzen mit einer Säure ausgewählt sind.

4. Zusammensetzung nach Anspruch 2, **dadurch gekennzeichnet, dass** die Indolderivate unter 4-Hydroxyindolin, 6-Hydroxyindolin, 6-Aminoindolin, 5,6-Dihydroxyindolin und ihren Additionssalzen mit einer Säure ausgewählt sind.

5. Zusammensetzung nach Anspruch 1, **dadurch gekennzeichnet, dass** die Benzimidazolderivate unter den Verbindungen der folgenden Formel (II) und ihren Additionssalzen mit einer Säure ausgewählt sind: 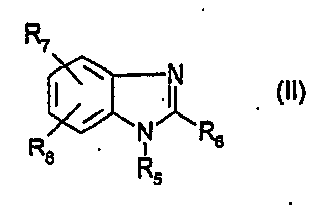 worin bedeuten:
R₅ ein Wasserstoffatom oder eine Alkyl(C₁₋₄)gruppe,
R₆ ein Wasserstoffatom, eine AlkylC₁₋₄)gruppe oder eine Phenylgruppe,
R₇ eine Hydroxygruppe, eine Aminogruppe oder eine Methoxygruppe,
R₈ ein Wasserstoffatom, eine Hydroxygruppe, eine Methoxygruppe oder eine Alkyl(C₁₋₄)gruppe;
mit der Maßgabe, dass:
- sich R₇ in 4-Stellung befindet, wenn R₇ eine Aminogruppe bedeutet,
- sich R₈ in 7-Stellung befindet, wenn sich R₇ in 4-Stellung befindet,
- sich R₈ in 6-Stellung befindet, wenn R₇ sich in 5-Stellung befindet.

6. Zusammensetzung nach Anspruch 5, **dadurch gekennzeichnet, dass** die Benzimidazolderivate der Formel (II) unter 4-Hydroxybenzimidazol, 4-Aminobenzimidazol, 4-Hydroxy-7-methylbenzimidazol, 4-Methoxy-2-methylbenzimidazol, 1-Butyl-4-hydroxybenzimidazol, 4-Amino-2-methylbenzimidazol, 5,6-Dihydroxybenzimidazol, 5-Hydroxy-6-methoxybenzimidazol, 4,7-Dihydroxybenzimidazol, 4,7-Dihydroxy-1-methylbenzimidazol, 4,7-Dimethoxybenzimidazol, 5,6-Dihydroxy-1-methylbenzimidazol, 5,6-Dihydroxy-2-methylbenzimidazol, 5,6-Dimethoxybenzimidazol und ihren Additionssalzen mit einer Säure ausgewählt sind.

7. Zusammensetzung nach Anspruch 1, **dadurch gekennzeichnet, dass** die Benzomorpholinderivate unter den Verbindungen der folgenden Formel (III) und ihren Additionssalzen mit einer Säure ausgewählt sind: 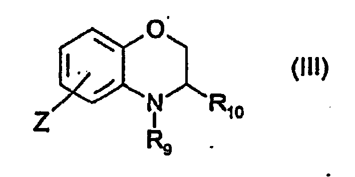 worin bedeuten:
R₉ und R₁₀, die gleich oder verschieden sind, ein Wasserstoffatom oder eine Alkyl(C₁₋₄)gruppe,
Z eine Hydroxygruppe oder eine Aminogruppe.

8. Zusammensetzung nach Anspruch 7, **dadurch gekennzeichnet, dass** die Benzomorpholinderivate der Formel (III) unter 6-Hydroxy-1,4-benzomorpholin, N-Methyl-6-hydroxy-1,4-benzomorpholin, 6-Amino-1,4-benzomorpholin und ihren Additionssalzen mit einer Säure ausgewählt sind.

9. Zusammensetzung nach Anspruch 1, **dadurch gekennzeichnet, dass** die Sesamolderivate unter den Verbindungen der folgenden Formel (IV) und ihren Additionssalzen mit einer Säure ausgewählt sind: 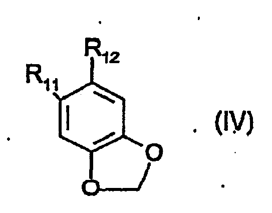 worin bedeuten:
R₁₁ eine Hydroxygruppe, eine Aminogruppe, eine Alkyl(C₁₋₄)aminogruppe, eine Monohydroxyalkyl(C₁₋₄)aminogruppe oder eine Polyhydroxyalkyl(C₂₋₄)aminogruppe,
R₁₂ ein Wasserstoffatom oder ein Halogenatom oder eine Alkoxy(C₁₋₄)gruppe.

10. Zusammensetzung nach Anspruch 9, **dadurch gekennzeichnet, dass** die Sesamolderivate der Formel (IV) unter 2-Brom-4,5-methylendioxyphenol, 2-Methoxy-4,5-methylendioxyanilin, 2-(β-Hydroxyethyl)amino-4,5-methylendioxybenzol und ihren Additionssalzen mit einer Säure ausgewählt sind.

11. Zusammensetzung nach Anspruch 1, **dadurch gekennzeichnet, dass** die Pyrazolo-azolderivate unter 2-Methyl-pyrazolo-[1,5-b]-triazol, 2-Ethyl-pyrazolo-[1,5-b]-1,2,4-triazol, 2-Isopropylpyrazolo-[1,5-b]-1,2,4-triazol, 2-Phenyl-pyrazolo-[1,5-b]-1,2,4-triazol, 2,6-Dimethyl-pyrazolo-[1,5-b]-1,2,4-triazol, 7-Chlor-2,6-dimethyl-pyrazolo-[1,5-b]-1,2,4-triazol, 3,6-Dimethyl-pyrazolo-[3,2-c]-triazol, 6-Phenyl-3-methylthiopyrazol-[3,2-c]-1,2,4-triazol, 6-Amino-pyrazolo-[1,5-a]-benzimidazol und ihren Additionssalzen mit einer Säure ausgewählt sind.

12. Zusammensetzung nach Anspruch 1, **dadurch gekennzeichnet, dass** die Pyrrolo-azolderivate unter 5-Cyano-4-ethoxycarbonyl-8-methyl-pyrrolo-[1,2-b]-1,2,4-triazol, 5-Cyano-8-methyl-4-phenylpyrrolo-[1,2-b]-1,2,4-triazol, 7-Amido-6-ethoxycarbonyl-pyrrolo-[1,2-a]-benzimidazol und ihren Additionssalzen mit einer Säure ausgewählt sind.

13. Zusammensetzung nach Anspruch 1, **dadurch gekennzeichnet, dass** die Imidazolo-azolderivate unter 7,8-Dicyano-imidazolo-[3,2-a]-imidazol, 7,8-Dicyano-4-methyl-imidazolo-[3,2-a]-imidazol und ihren Additionssalzen mit einer Säure ausgewählt sind.

14. Zusammensetzung nach Anspruch 1, **dadurch gekennzeichnet, dass** die Pyrazolo-pyrimidinderivate unter Pyrazolo-[1,5-a]-pyrimidin-7-on, 2,5-Dimethyl-pyrazolo-[1,5-a]-pyrimidin-7-on, 2-Methyl-6-ethoxycarbonyl-pyrazolo-[1,5-a]-pyrimidin-7-on, 2-Methyl-5-methoxymethyl-pyrazolo-[1,5-a]-pyrimidin-7-on, 2-*tert-*Butyl-5-trifluormethyl-pyrazolo-[1,5-a]-pyrimidin-7-on, 2,7-Dimethyl-pyrazolo-[1,5-a]-pyrimidin-5-on und ihren Additionssalzen mit einer Säure ausgewählt sind.

15. Zusammensetzung nach Anspruch 1, **dadurch gekennzeichnet, dass** die Pyrazolin-3,5-dionderivate unter 1,2-Diphenylpyrazolin-3,5-dion, 1,2-Diethyl-pyrazolin-3,5-dion und ihren Additionssalzen mit einer Säure ausgewählt sind.

16. Zusammensetzung nach einem der vorhergehenden Ansprüche, **dadurch gekennzeichnet, dass** die Oxidationsbase oder die Oxidationsbasen unter den para-Phenylendiaminen, Doppelbasen, para-Aminophenolen, ortho-Aminophenolen und heterocyclischen Oxidationsbasen ausgewählt sind.

17. Zusammensetzung nach einem der vorhergehenden Ansprüche, **dadurch gekennzeichnet, dass** die Oxidationsbase oder die Oxidationsbasen 0,0005 bis 12 Gew.-% des Gesamtgewichts der Farbmittelzusammensetzung ausmachen.

18. Zusammensetzung nach Anspruch 17, **dadurch gekennzeichnet, dass** die Oxidationsbase oder die Oxidationsbasen 0,005 bis 6 Gew.-% des Gesamtgewichts der Farbmittelzusammensetzung ausmachen.

19. Zusammensetzung nach einem der vorhergehenden Ansprüche, **dadurch gekennzeichnet, dass** das 1,3-Bis(β-hydroxyethyl)-amino-2-methylbenzol und/oder das oder die Additionssalze dieser Verbindung mit einer Säure 0,001 bis 10 Gew.-% des Gesamtgewichts der Farbmittelzusammensetzung ausmachen.

20. Zusammensetzung nach Anspruch 19, **dadurch gekennzeichnet, dass** das 1,3-Bis(β-hydroxyethyl)amino-2-methylbenzol und/oder das oder die Additionssalze der Verbindung mit einer Säure 0,01 bis 5 Gew.-% des Gesamtgewichts der Farbmittelzusammensetzung ausmachen.

21. Zusammensetzung nach einem der vorhergehenden Ansprüche, **dadurch gekennzeichnet, dass** der heterocyclische Kuppler oder die heterocyclischen Kuppler 0,0001 bis 10 Gew.-% des Gesamtgewichts der Farbmittelzusammensetzung ausmachen.

22. Zusammensetzung nach Anspruch 21, **dadurch gekennzeichnet, dass** der heterocyclische Kuppler oder die heterocyclischen Kuppler 0,005 bis 5 Gew.-% des Gesamtgewichts der Farbmittelzusammensetzung ausmachen.

23. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

24. Verfahren zum Färben von Keratinfasern und insbesondere menschlichen Keratinfasern wie Haaren, **dadurch gekennzeichnet, dass** auf die Fasern mindestens eine Farbmittelzusammensetzung, wie sie in einem der Ansprüche 1 bis 23 definiert ist, aufgetragen wird und die Farbe bei einem sauren, neutralen oder alkalischen pH-Wert mit einem Oxidationsmittel gebildet wird, das bei der Anwendung zu der Farbmittelzusammensetzung gegeben wird oder das in einer oxidierenden Zusammensetzung enthalten ist, die gleichzeitig oder getrennt davon anschließend aufgebracht wird.

25. Verfahren nach Anspruch 24, **dadurch gekennzeichnet, dass** das in der oxidierenden Zusammensetzung enthaltene Oxidationsmittel unter Wasserstoffperoxid, Harnstoffperoxid, Alkalimetallbromaten, Salzen von Persäuren, wie Perboraten, Percarbonaten und Persulfaten, Persäuren und Enzymen ausgewählt ist.

26. Vorrichtung mit mehreren Abteilungen oder "Kit" zum Färben mit mehreren Abteilungen, wobei eine erste Abteilung eine Farbmittelzusammensetzung, wie sie in einem der Ansprüche 1 bis 23 definiert ist, und eine zweite Abteilung eine oxidierende Zusammensetzung enthält.
